Europäisches Patentamt

European Patent Office

Office européen des brevets

(11) Publication number: **0 268 066**
**A2**

# EUROPEAN PATENT APPLICATION

(21) Application number: 87114868.0

(22) Date of filing: **12.10.87**

(51) Int. Cl.4: **A61K 37/43** , A61K 31/557

(30) Priority: **17.10.86 US 920483**

(43) Date of publication of application:
**25.05.88 Bulletin 88/21**

(84) Designated Contracting States:
**AT BE CH DE ES FR GB GR IT LI LU NL SE**

(71) Applicant: **SYNTEX (U.S.A.) INC.**
**3401 Hillview Avenue**
**Palo Alto, California 94304(US)**

(72) Inventor: **Vickery, Brian H.**
**20279 Carol Lane**
**Saratoga California 95070(US)**

(74) Representative: **Barz, Peter, Dr. et al**
**Patentanwälte Dipl.-Ing. G. Dannenberg Dr.**
**P. Weinhold, Dr. D. Gudel Dipl.-Ing. S.**
**Schubert, Dr. P. Barz Siegfriedstrasse 8**
**D-8000 München 40(DE)**

(54) **Fertility control and uterine therapy in dogs with luteinizing hormone releasing hormone antagonists.**

(57) Methods of controlling fertility and treating hormonally mediated uterine infections in female dogs by administering an effective amount of an LHRH antagonist.

EP 0 268 066 A2

# FERTILITY CONTROL AND UTERINE THERAPY IN DOGS WITH LUTEINIZING HORMONE RELEASING HORMONE ANTAGONISTS

This invention relates to methods of controlling fertility and treating uterine infection in female dogs. In particular, the invention relates to methods of interrupting heat and pregnancy and to methods of treating uterine infections in dogs using luteinizing hormone releasing hormone (LHRH) antagonists.

There are several known methods of controlling fertility in dogs. However, there is no available method which is reversible, readily administrable, and free of significant undesirable side effects.

The most common and popular method of preventing pregnancy in the bitch is by ovariohysterectomy. However, this method has the disadvantage of being irreversible and is therefore unacceptable in breeding stock. Prostaglandins will terminate pregnancy if administered in mid-gestation or later. However, the abortive action is accompanied by intolerable side effects including emesis, diarrhea and changes in thermoregulatory and cardiovascular parameters (Vickery & McRae Biol. Reprod. 22:438-442, 1980). The cardiovascular side effects in particular result in very low therapeutic ratios. Estrogens have also been used very shortly after mating as an interceptive method of preventing pregnancy ("mis-mating"), (Bower et al., JAVMA 186: 733-738, 1985). However, concerns over induction of nymphomania, and problems of carcinogenesis and cardiovascular complication, particularly thrombogenesis, leave these steroids contraindicated for this use (Olsen et al., Compend. Cont. Educ. 8: 87-92, 1986.

Progestational steroids were used as long term estrus suppressants (Harris and Wotchuk Am. J. Vet. Res. 24:1003-1006, 1963). Unfortunately, the requirement of dialy oral administration, and unacceptable side effects, specifically pyometritis, endometritis (Anderson, Gillmore and Schnelle J. Am. Vet. Med Assoc. 146:1311-1316, 1965) and mammary nodules have relegated their present utility merely to short courses of treatment for temporary postponement of estrus (Burke and Reynolds J. Am. Med Assoc. 167:285-287, 1975). An androgenic steroid, Mibolerone®, is available commercially for long term estrus suppression in bitches, but must be administered daily in the dog's food, and demonstrates high interbreed variations in dosage requirements and masculinizing effects on hair quality. The affectiveness of this method is also dependent upon the dog consuming all of its food. (Sokolowski and Geng Am. J. Vet. Res. 38:1371-1376, 1977).

Luteinizing hormone (LH) and follicle stimulating hormone (FSH) are the two gonadotropic hormones which regulate gametogenesis and gonadal steroidogenesis and are therefore responsible for reproductive cyclicity and for all or part of the course of gestation in mammals. Luteinizing hormone releasing hormone (Various synonyms include LH-RH; GnRH; LH/FSH-RH, but LHRH will be used herein) controls the synthesis and release of LH and FSH. LHRH occurs naturally as a decapeptide comprised of naturally occurring amino acids (which have the L-configuration except for the achiral amino acid glycine). Its sequence is as follows:

$$\text{(pyro) Glu-His-Trp-Ser-Tyr-Gly-Leu-Arg-Pro-Gly-NH}_2$$
$$1 \quad 2 \quad 3 \quad 4 \quad 5 \quad 6 \quad 7 \quad 8 \quad 9 \quad 10$$

Agonist analogs of LHRH have been tested as fertility controlling agents in dogs. However, it was found that estrus suppression could only be achieved by continuous daily administration, and that the agonists were not effective to terminate pregnancy. See McRae, G.I., et al., J. Reprod. Fert. 74:389-397, 1985.

Analogs have been prepared which are competitive antagonists to LHRH; all of which require deletion or replacement of the histidine residue at position 2 (Vale, W., et al, Science, 176: 933 ,1972).

It has also been shown that adding a modification at the 6 position enhances the antagonist activity of the 2-modified analogs (Beattie, C. W., et al, J. Med. Chem., 18: 1247, 1975; Rivier, J., et al, Peptides 1976 p. 427, Editions de l'Universite de Bruxelles, Belgium, 1976).

Against the background of these two major alterations, which result in a potent series of LHRH antagonists, additional increments in antagonist activity may be had by modifying positions 1, 3 and/or 10 in the already 2, 6-modified peptide. See for example, Coy, D. H., et al Peptides 1976, p. 462, Editions de l'Universite de Bruxelles, Belgium, 1976; Rivier, J. E., et al, Life Sci. 23: 869, 1978. It has also been shown that N-acylation of the amino acid at position 1 is helpful. See for example, Coy, D. H., et al, Peptides. - Structure and Biological Function p. 775, Pierce Chemical Co., 1979. In addition, hydrophobic substitutions at position 7 have been found to be useful. See for example, Folkers, K. et al, Biochem. Biophys. Res. Commun., 123, 1221-1226,1984.

Additionally, (N-Ac-D-p-Cl-Phe[1], D-p-Cl-Phe[2], D-Trp[3], D-Arg[6], D-Ala[10])LHRH has been reported by D.H.

Coy et al., Endocrinology , 110, 1445 (1982). A series of highly potent LHRH antagonists were disclosed in European Patent Application No. 83303343.4, including [N-Ac-D-Nal(2)[1], D-p-Cl-Phe[2], D-Trp[3], D-Deh[6], D-Ala[10]]LHRH. These compounds were disclosed as having a range of effects and utilities in mammals, including contraception, ovulation inhibition, estrus suppression and early (first trimester) termination of pregnancy, among various other uses.

However while one might expect LHRH antagonists to work equally well to control fertility in all mammals,such is in fact not the base. Species variability has confounded attempts to develop practical methods of fertility control for many animals. In the regulation and function of the corpus luteum, adaptations appear to have arisen variously from specie to specie. In some species such as rodents (mice, rats) luteinizing hormone serves as the luteotropin until about day 12 of gestation; in other such as dogs and cattle, prolactin plays such a role, while in some species, notably primates, the feto-placental unit is involved. During pregnancy, the pituitary seems essential in some animals, but is clearly not in others. Thus, luteal function may be autonomous, or it may be regulated in part by placental luteotropin, as for example in primates including women.

Furthermore, the interactions and regulation of gonadotropins are highly complex, and as yet poorly understood. While the implications of gonadotropin regulation have prompted the synthesis of hundreds of analogs of LHRH, as described above, paradoxical effects have been observed which have rendered the use of these analogs difficult and unpredictable. LHRH administration is known to stimulate ovulation in women and some animals. Paradoxically, prolonged use of high concentrations of LHRH has caused ovarian and uterine atrophy.

For example, the developing feto-placental unit of certain mammalian species is known to exercise autonomous control of the pregnancy by independently eliminating the requirement of LH and FSH, thus thwarting the effects of an LHRH antagonist administered to terminate the pregnancy. This is particularly noted in the primate, including women, in which treatment with an LHRH antagonist during the non pregnant luteal phase results in suppression of progesterone levels and premature menstruation, but treatment during early pregnancy or in the face of HCG supplementation is ineffective (Fraser, et al., J. Endocrinol. 104, R1, 1985; Siler-Khodr , et al. Fertil. Steril. 41, 448, 1984; Mais, et al. J. Clin Endocrinol. Metab. 62m 1250, 1986). LHRH antagonists are also ineffective to terminate pregnancy in cats.

In contrast to many mammals, including primates, rats and rabbits, in which the effects of LHRH antagonists have been reported, the cyclic periods of fertility in cats and dogs are governed not only by ovulation but also by cyclic sexual behavior, estrus or heat. In dogs, heat is an approximately bi-annual period of breeding behavior which precedes ovulation. The exact time of onset of heat is unpredictable, and heat itself is marked by sexual receptivity, pheromone production and vaginal discharge, and elicits aggressive sexual behavior by proximate male dogs.

Thus there is a need for a well tolerated, reversible, easily administerable non-steroidal method of controlling fertility in female dogs. Such a method would ideally be capable of administration to interrupt heat and estrus, thereby terminating breeding behavior, pheromone production and vaginal discharge. To facilitate breeding of a female dog, the ideal method of fertility control would be capable of administration to closely control the timing of breeding behavior. Additionally, such a method would be capable of administration to terminate pregnancy. Because the exact data of mating is frequently not known, the ideal method would be effective in terminating gestation when administered at any time after mating.

This invention provides a method of controlling fertility in female dogs by administering an effective amount of an LHRH antagonist. Thus, one aspect of the invention is a method of terminating estrus by administration of an effective amount of an LHRH antagonist.

An important aspect of the invention is the surprising control of estrus timing which is achieved by administration of an LHRH antagonist to a female dog at any time during pro-estrus; diestrus occurs within two days, and pro-estrus returns on or about 26 days later. Accordingly, another aspect of the invention is a method of controlling the time of estrus in a female dog by administering an LHRH antagonist at a chosen time during a pro-estrus period.

The present invention also provides a means of terminating pregnancy in a dog at any time after mating. Unexpectedly, studies with LHRH antagonists have now shown that the antagonists work particularly well to terminate pregnancy in dogs, but are ineffective in terminating pregnancy in cats. Accordingly, a further aspect of the invention provides a method of terminating pregnancy in a dog by administering an effective amount of an LHRH antagonist.

Administration at any time prior to day 14 of gestation of a single high dose of an LHRH antagonist will bring about termination of pregnancy at about day 18 of gestation. Similarly, later administration of a single high dose of an LHRH antagonist will cause termination of pregnancy within a few days. Accordingly, an aspect of the invention is a method of terminating pregnancy by administration of a single high dose of an LHRH antagonist.

Alternatively, co-administration of a synergistic combination of low, independently ineffective doses of an LHRH antagonist and a prostaglandin or prostaglandin analog at any time from just after mating until the end of gestation will terminate pregnancy. Therefore, a further aspect of the invention is a method of terminating pregnancy at any time by co-administration of an LHRH antagonist and a prostaglandin or prostaglandin analog.

Termination of pregnancy on or after about 14 days of gestation can also be achieved by administration, continuously or daily for a period of about 4 to 8 days, of a low dose of an LHRH antagonist, or by administration of a single dose of an amount of LHRH antagonist equivalent to 1 to 2 times the sum of the daily doses. Therefore, another aspect of the invention is a method of terminating pregnancy in a dog on or after about 14 days of gestation by administering either a low daily dose of an LHRH antagonist over a period of about 4 to 8 days, or a single dose of an LHRH antagonist equivalent to about 1 to 2 times the sum of the daily doses.

This invention also provides a method of treating hormonally mediated uterine infections in dogs, particularly pyometritis and endometritis, by administration of an effective amount of a combination of an LHRH antagonist and a prostaglandin or prostaglandin analog.

At the present time, the available treatments for pyometritis are prostaglandin therapy and ovariohysterectomy. However, prostaglandin therapy, at the dosage levels required, presents significant undesirable side effects requiring hospitalization, and ovariohysterectomy renders the animal irreversibly sterile. The present invention provides effective treatment for pyometritis, without the serious side effects of conventional prostaglandin therapy or the ireversibility of ovariohysterectomy.

Further aspects of the invention will become apparent from the Detailed Description and Preferred Embodiments and include pharmaceutical compositions for controlling fertility and treating hormonally mediated uterine infections in dogs.

## ABBREVIATIONS AND DEFINITIONS

For convenience in describing the invention, the conventional abbreviations for the various common amino acids which make up the LHRH antagonists of this invention are used as recommended by the IUPAC-IUB Commission on Biochemical Nomenclature, Biochemistry, 11, 1726 (1972). All peptide sequences mentioned herein are written according to the generally accepted convention whereby the N-terminal amino acid is on the left and the C-terminal amino acid is on the right.

Certain other abbreviations will be useful in describing the invention. The preferred LHRH antagonists of the present invention include amino acid residues which do not occur in nature. Particularly common non naturally occurring amino acids residues incorporated in the preferred LHRH antagonists of this invention are:

| Amino acid residue | Abbreviation |
|---|---|
| 3-(2-naphthyl)-D-alanyl | D-Nal(2) |
| 3-(p-fluorophenyl)-D-alanyl | D-p-F-Phe |
| 3-(p-chlorophenyl)-D-alanyl | D-p-Cl-Phe |
| 3-(p-bromophenyl)-D-alanyl | D-p-Br-Phe |
| 3-(4-(trifluoromethylphenyl)-D-alanyl | D-Ptf |
| $N^G,N^{G'}$-diethyl-D-homoarginine | D-Deh |
| $N^G,N^{G'}$-bis(2,2,2-trifluoroethyl)-D-homoarginine | D-FDeh |
| 3-(3-pyridyl)-D-alanyl | D-Pal(3) |

Those amino acids that are the D-configuration of naturally occurring amino acids will be so noted.

The abbreviation "N-Ac-" refers specifically to the N-acetyl amino acid residue in conformance with generally accepted nomenclature.

As a further convenience, since the amino acid sequence of LHRH has been shown to be

$$(pyro)Glu-His-Trp-Ser-Tyr-Gly-Leu-Arg-Pro-Gly-NH_2,$$

$$1 \quad 2 \quad 3 \quad 4 \quad 5 \quad 6 \quad 7 \quad 8 \quad 9 \quad 10$$

nona-, and decapeptides in which the amino acid residues at particular places in the sequence have been replaced by other amino acid residues or other moieties are abbreviated by showing the nature of the substitution, superscribed by the location, followed by LHRH as the parent.

Thus, for example, the sequence,

$$(pyro)Glu-D-p-F-Phe-Trp-Ser-Tyr-D-Deh-Leu-Arg-Pro-GlyNH_2$$

$$1 \quad 2 \quad 3 \quad 4 \quad 5 \quad 6 \quad 7 \quad 8 \quad 9 \quad 10$$

in which the Gly at position 6 has been replaced by D-Deh and the His at position 2 has been replaced by D-p-F-Phe, is represented [D-p-F-Phe[2], D-Deh[6]]LHRH; and the sequence

$$N-Ac-D-Nal(2)-D-p-Cl-Phe-D-Trp-Ser-Tyr-D-Deh-$$

$$1 \quad\quad 2 \quad\quad 3 \quad 4 \quad 5 \quad\quad 6$$

$$-Leu-Arg-Pro-D-Ala$$

$$7 \quad 8 \quad 9 \quad 10$$

is represented: [N-Ac-D-Nal(2)[1], D-p-Cl-Phe[2], D-Trp[3], D-Deh[6], D-Ala[10]]LHRH.

As used herein, the term "pharmaceutically acceptable salts" refers to salts that retain the desired biological activity of the parent compound and do not impart any undesired toxicological effects. Examples of such salts are:

(a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, naphthalenesulfonic acids, naphthalenedisulfonic acids, polygalacturonic acid;

(b) salts with polyvalent metal cations such as zinc, calcium, bismuth, barium, magnesium, aluminum, copper, cobalt, nickel, cadmium, and the like; or with an organic cation formed from N,N'-dibenzylethylene-diamine or ethylenediamine; or

(c) combinations, of (a) and (b), e.g., a zinc tannate salt and the like.

"Halo lower alkyl" refers to a lower alkyl radical substituted with halo groups, especially those having one, two or three halo groups on the $\omega$-carbon. The halo group may be fluoro, chloro or bromo. This group is exemplified by trifluoromethyl, 2,2,2-trifluoroethyl, 3,3,3-trifluorpropyl, 2,2,2-trichloroethyl and the like.

The term "optionally substituted" meas that the stated optional substitution may or may not occur, and that both circumstances are within the intended scope of the invention.

As used herein, the term "treatment" covers any treatment of a hormonally mediated uterine infection in a dog, and include:

(i) inhibiting the disease, i.e. arresting its development; and or

(ii) relieving the disease, i.e. causing regression of the disease, or curing the disease.

The term "gestation" refers to the development of pregnancy. The first day, or "day 1" of gestation is defined as the first day of diestrus, as determined by vaginal cytology.

## PREFERRED LHRH ANTAGONISTS

LHRH antagonists suitable for use in the present invention are nona-and decapeptide analogs of naturally occurring LHRH which have potent LHRH antagonist activity, and the pharmaceutically acceptable acid addition salts thereof. One preferred class of compounds are those defined by [N-Ac-D-Nal(2)[1], D-p-Halo-Phe[2], D-Trp[3], D-Deh[6], D-Ala[10]]LHRH, wherein the D-Deh[6] substituent is optionally substituted with a halo lower alkyl group; these are described in U.S. Patent Nos. 4,481,190, 4,581,169, and 4,667,014. Other preferred LHRH antagonists include those described in U.S. Patent Nos. 4,419,347, 4,341,767, and 4,690,916.

Particularly preferred LHRH antagonists suitable for use in the present invention include:

[N-Ac-D-Nal(2)[1], D-p-Cl-Phe[2], D-Trp[3], D-Deh[6], D-Ala[10]]LHRH;

[N-Ac-D-Nal(2)[1], D-p-F-Phe[2], D-Trp[3], D-Deh[6] D-Ala[10]]LHRH;

[N-Ac-D-Nal(2)[1], D-p-Cl-Phe[2], D-Trp[3], D-FDeh[6], D-Ala[10]]LHRH; and

[N-Ac-D-Nal(2)[1], D-p-Cl-Phe[2], D-Trp[3], D-Den[6], aza-Gly[10]]LHRH.

Other preferred LHRH antagonists include:

[N-Ac-$\Delta^3$-Pro[1], D-p-F-Phe[2], D-Trp[3]m[6], NMe-Leu[7]]LHRH;

[N-Ac-$\Delta^3$-Pro[1], D-p-F-Phe[2], D-Nal(2)[3,6]]LHRH;

[N-Ac-Pro[1], D-p-F-Phe[2], D-Nal(2)[3,6,]]LHRH;

[N-Ac-Ala[1], D-p-F-Phe[2], D-Trp[3,6]]LHRH;

[N-Ac-D-Trp[1], D-p-Cl-Phe[2], D-Trp[3], D-Phe[6], D-Ala[10]]LHRH;

[N-Ac-D-Trp[1], D-p-Cl-Phe[2], D-Trp[3], D-Arg[6], D-Ala[10]]LHRH;

[N-Ac-D-p-Cl-Phe[1], D-p-Cl-Phe[2], D-Trp[3], D-Arg[6], D-Ala[10]]LHRH;

[N-Ac-D-Nal(2)[1], D-p-F-Phe[2], D-Trp[3], D-Arg[6]]LHRH;

[N-Ac-D-Nal(2)[1], D-p-F-Phe[2], D-Trp[3], oCl-Phe[5], D-Arg[6]]LHRH;

[N-Ac-D-p-Br-Phe[1], D-p-Cl-Phe[2], D-Trp[3], D-Arg[6], D-Ala[10]]LHRH;

[N-Ac-D-Nal(2)[1], D-p-Cl-Phe[2], D-Trp[3], D-Arg[6], D-Ala[10]]LHRH;

[N-Ac-D-Nal(2)[1], D-p-Cl-Phe[2], D-Pal(3)[3], D-Arg[6], D-Ala[10]]LHRH;

[N-Ac-D-Nal(2)[1], D-p-Cl-Phe[2], D-Pal(3)[3], D-Arg[6], D-Trp[7], D-Ala[10]]LHRH;

[N-Ac-D-Nal(2)[1], D-p-Cl-Phe[2], D-Pal(3)[3], L-Arg[6], D-Glu(p-MeO-Phe)[6], D-Ala[10]]LHRH;

[N-Ac-D-Nal(2)[1], D-$\alpha$-Me-p-Cl-Phe[2], D-Pal(3)[3], N-iso-propyl-Lys[5,8], D-Tyr[6], D-Ala[10]]LHRH;

[N-Ac-D-Nal(2)[1], D-$\alpha$Me-p-Cl-Phe[2], D-Trp[3], Arg[5], D-Tyr[6], D-Ala[10]]LHRH; and

[N-Ac-D-Nal(2)[1], D-$\alpha$Me-p-Cl-Phe[2], D-Pal(3)[3], D-Arg[6], N-iso-propyl-Lys[8], D-Ala[10]]LHRH.

The prostaglandins and prostaglandin analogs which are suitable for use in this invention are those prostaglandin compounds which have luteolytic and uterotonic activity. A large number of potent prostaglandin analogs have been synthesized which possess both luteolytic and uterotonic activity and are suitable for use in this invention. Most of these are analogs of $PGF_\alpha$, $PGE_1$ and $PGE_2$. A review of the PGE and PGF analogs is provided by J. Muchowski in the Handbook of the Eicosanoids: Prostaglandins and Related Lipids, Volume 2, edited by A.L. Willis, CRC Press, Boca Raton, Florida (In Press, 1987), which identifies many potent luteolytic/uterotonic agents. Among the analogs of $PGF_\alpha$, of greatest interest are analogs of $PGF_{1\alpha}$ and $PGF_{2\alpha}$. Specific preferred prostaglandin analogs of the present invention include the following compounds, and their alkyl esters having one to twelve carbon atoms:

Methyl 5-Oxo-9$\alpha$,11$\alpha$,15$\alpha$-trihydroxy-17-phenyl-$\omega$-trinorprosta-13-trans-enoate;

Methyl 9$\alpha$,11$\alpha$-15$\epsilon$-trihydroxy-15$\epsilon$-methylprosta-4,5-13-tran s-trienoate (Prostalene);

9$\alpha$,11$\alpha$,15$\alpha$-Trihydroxy-15$\beta$-methylprosta-5-cis-1 3-trans-trienoic acid (Carbaprost);

Methyl 9$\alpha$,11$\alpha$,15$\alpha$-Trihydroxy-15-benzo(b)-furan 2-yl-$\omega$-pentanorprosta-5-cis-13-trans-dienoate;

Methyl 9$\alpha$,11$\alpha$,15$\alpha$-Trihydroxy-16-cyclohexyl-$\omega$-tetranorprosta-5-cis-13-ynoicacid; (Alfaprostol)

9$\alpha$,11$\alpha$,15$\alpha$-Trihydroxy-16-(3-chlorophenoxy)-$\omega$-tetranorprosta-5-cis-13-trans-dienoic acid (Estrumate);

9$\alpha$,11$\alpha$,15$\alpha$-Trihydroxy-16-(3-trifluoromethylphenoxy)-$\omega$-tetranorprosta-5-cis-13-trans-dienoic acid (Equimate);

9$\alpha$,11$\alpha$,15$\alpha$-Trihydroxy-16-(3-thienyloxy)-$\omega$-tetranorprosta-5-cis-13-trans-dienoic acid (Tiaprost);

Methyl 9$\alpha$,11$\alpha$,15$\alpha$-Trihydroxy-16-(3-chlorophenoxy)-$\omega$-tetranorprosta-2-trans-5-cis-13-trans-trienoate (Delprostenate);

Methyl ·9$\alpha$,11$\alpha$,15$\alpha$-Trihydroxy-16-(3-trifluoromethylphenoxy)-$\omega$-tetranorprosta-2-trans-5-cis-13-trans-dienoate (ONO-995);

9$\alpha$,11$\alpha$,15$\alpha$-Trihydroxy-16-(3-chlorophenoxy)-$\omega$-tetranor-4-cis-13-trans-dienoic acid;

Methyl 9$\alpha$,11$\alpha$,15$\alpha$-trihydroxy-16-phenoxy-$\omega$-tetranorprosta-4,5-13-trans-trienoate (Fenprostalene);

9$\alpha$,11$\alpha$,15$\alpha$-Trihydroxy-13-thia-16-(3-chlorophenoxy)-$\omega$-tetranorprosta-5-cis-enoic acid;

9$\alpha$,11$\alpha$-Dihydroxy-15-keto-16-phenoxy-$\omega$-tetranorprosta-5-cis -13-trans-dienoic acid 15-ethylene ketal;

Methyl 9$\alpha$,11$\alpha$,15$\alpha$-trihydroxy-17-(2-chlorophenyl)-$\omega$-trinorprosta-5-cis-13-trans-16-ynoate;

Methyl 9$\alpha$,11$\alpha$,15$\alpha$-trihydroxy-17-(2-furyl)-$\omega$-trinorprosta-5-cis -13-trans-16-ynoate

Methyl 9$\alpha$,11$\alpha$,15$\alpha$-trihydroxy-16$\epsilon$,17$\epsilon$-methano-17-phenyl-$\omega$-trinorprosta-5-cis-13-trans-dienoate;

Methyl 9$\alpha$,11$\alpha$,15$\alpha$-trihydroxy-17-keto-17-phenyl-$\omega$-trinorprosta-5-cis-13-trans-dienoate.

9-Methylene-11$\alpha$,15$\alpha$-dihyroxy-16,16-dimethylprosta -5-cis-13-trans-dienoic acid (Meteneprost);

Methyl 5-oxo-9$\alpha$,11$\alpha$-dihydroxy-17-phenyl-$\omega$-trinorprosta-13-trans -enoate; and

N-methanesulfonyl-9-keto-11$\alpha$,15$\alpha$-dihydroxy-16-phenoxy-$\omega$-tetranorprosta-5-cis-13-trans-dienoamide (Sulprostone).

Particularly preferred prostaglandin analogs for use in this invention are Prostalene and Fenprostalene and their alkyl esters having one to twelve carbon atoms, more particularly the alkyl esters having one to four carbon atoms, especially the isopropyl esters. These compounds are described in U.S. Patent Nos. 3,879,438 and 3,985,791, respectively. Additional pharmaceutical compositions containing these compounds are described in U.S. Pat. No. 4,389,414.

Another preferred compound is 16(s)-Amino-PGF$_{2\alpha}$methyl ester acetate salt, which is described in Prostaglandins, Feb. 1985, Vol. 29, No. 2, pp. 303-312. Still other preferred prostaglandin analogs include the 9-halo prostaglandin analogs such as those described in U.S. Pat. No. 4,444,788 and German Offenlegunsschrift DE 34 14 509 Al.

UTILITIES AND METHODS OF ADMINISTRATION

The methods provided by this invention of controlling fertility and treating uterine infections in dogs may be administered by any of the accepted modes of administration of pharmacologic agents to dogs. These methods include oral, parenteral, rectal, buccal, nasal and aerosol, and otherwise systemic forms. However, the preferred mode of administration is parenteral, preferably subcutaneously or intramuscularly, either by injection of a parenteral solution or suspension, or by injection, insertion or implantation of a parenteral sustained release or depot formulation. The active LHRH and prostaglandin analogs used in these methods are substantially more potent when administered parenterally than when administered by any other route; non-parenteral administration of an LHRH antagonist would require approximately 50 - 1000 times higher doses than parenteral administration, and non-parenteral administration of a prostaglandin analog would require approximately 2 - 10 times higher doses than those required for parenteral administration. Accordingly, the dosage ranges recommended herein for each of the various preferred methods of practicing the invention are for parenteral administration. Non-parenteral administration, while not preferred, may of course be utilized and is within the scope of the invention; suitable dosage ranges for non-parenteral administration will be approximately 50 - 1000 times the range stated herein for the LHRH antagonist, and about 2 - 10 times the range stated herein for the prostaglandin analog.

As described above, one aspect of the invention is the discovery that pregnancy in a dog can be terminated at any time after mating by administering a single low dose of about 0.5 - 2 mg/kg of an LHRH antagonist in combination with a single low dose of about 5-20 μg/kg of a prostaglandin analog. (Generally speaking, a low dose of an LHRH antagonist as described above would be independently ineffective in terminating pregnancy before about day 14 of gestation.) The LHRH antagonist and the prostaglandin analog may be co-administered in a single combined dosage form. In this method, as well as the other methods of terminating pregnancy, interrupting estrus and treating uterine infections, the dose ranges stated herein are for compounds within the potency range of the preferred compounds for use in this invention. If LHRH antagonists and prostaglandins having lower potencies are used, doses higher than those stated herein may be required, and are included within the scope of this invention.

An alternative method of terminating pregnancy is by administration of a single high dose of an LHRH antagonist in the range of about 8-20 mg/kg. The dose may be effectively administered at any time after mating, but termination of pregnancy will not occur before about 18 days of gestation.

Termination of pregnancy by dosing at any time on or after about 14 days of gestation can also be achieved by administration of a relatively low dose of an LHRH antagonist, in the range of about 50-350 μg/kg/day administered over a period of about 4-8 days. Specifically, the dose may be in the range 50-250 μg/kg/day. Alternatively, a single bolus dose of about 0.2-6 mg/kg of an LHRH antagonist will terminate a pregnancy when administered at any time after about 14 days gestation.

The invention also provides a method of inducing labor and delivery. If the LHRH antagonist is administered late in pregnancy close to term, preferably on or after day 60 of gestation, the pregnancy terminating activity of the antagonist will induce delivery. This may be particularly useful when a large litter is expected or other complications are present such that it would be advantageous to the bitch or offspring to induce delivery in the presence of qualified veterinary supervision.

Further aspects of the invention are methods of terminating estrus and heat, and of controlling the timing of the estrous period in dogs.

Termination of estrus may be achieved at any time during the estrous episode by daily administration for about 7-14 days of an effective amount of an LHRH antagonist, preferably, 50-350 μg/kg/day. Alternatively, a single injection of an amount equal to 1 to 2 times the sum of the daily doses, for example, a single injection of between 1500 μg/Kg to 4500 μg/Kg, preferably, 1500-2500 μg/Kg, is given at any time during an estrous episode, but preferably at the first signs of sanguineous vaginal discharge. Treatment by either method results in a rapid return to a diestrous state, as judged by disappearance of vaginal discharge, a regression of vaginal cytology, an unwillingness of the bitch to accept a stud male or to breed, and a disappearnce of attractiveness of the bitch to the male. In addition, ovulation does not occur.

The remarkable synchrony of the timing of return of estrus in bitches in which estrus has been terminated with an LHRH antagonist provides a very useful management tool for the breeder of dogs. Bitches come into season (heat) only 1-2 times per year without warning. In many cases, for commercial, or show breeding purposes, it is desirable to breed the bitch with a stud show breeding male who may be located at a distance geographical location. The present invention provides the ability to terminate an ongoing heat and have it return approximately 26 days later, thus allowing ample time to arrange an appointment, stud fees and transportation.

This invention also provides methods to treat hormonally mediated uterine infections, particularly pyometritis and endometritis. In the preferred method, the bitch is treated simultaneously with an LHRH angatonist and a prostaglandin analog. A single low dose of an LHRH antagonist, in the range of about 0.1-2 mg/kg, is co-administered with a single low dose, in the range of about 1-20 $\mu$g/kg, of a prostaglandin analog. The LHRH antagonist and prostaglandin analog may be combined in a single dosage form or pharmaceutical composition.

The dose of the prostaglandin analog required is directly related to the endogenous levels of progesterone, and as the plasma progesterone levels are precipitously decreased by the LHRH antagonist, therefore the therapeutic dosage requirement of the prostaglandins is reduced. Reduction of dosage of the prostaglandins leads in turn to a reduced incidence of side effects.

Surprisingly, the synergism between the LHRH antagonist and the prostaglandin is so great that the conventional prostaglandin-only treatment, which requires 4 times daily dosing for at least four days and may require a further course of treatment one month later. is reduced to a single injection each of the LHRH antagonist and the prostaglandin at substantially lower, non-toxic doses, with the additional advantage that no hospitalization is required.

Pharmaceutical compositions for use in this invention may comprise an active LHRH antagonist, or an active prostaglandin analog, or an active LHRH antagonist in combination with an active prostaglandin analog, in admixture with one or more pharmaceutically acceptable, non-toxic carriers and excipients. Such compositions may be prepared for use for parenteral administration (including subcutaneous, intramuscular or intravenous), particularly in the form of liquid solutions or suspensions; for use for vaginal or rectal administration, particularly in semisolid forms such as creams and suppositories; and for oral or buccal administration particularly in the form of tablets or capsules.

Depending on the intended mode of administration, the compositions may be in the form of solid, semi-solid or liquid dosage forms, such as, for example, tablets, suppositories, pills, capsules, powders, liquids, suspensions, or the like, preferably in unit dosage forms suitable for single administration of precise doses. In addition to one or more conventional carriers and/or excipients, the compositions may include other medicinal agents, pharmaceutical agents, adjuvants, and the like.

The parenteral pharmaceutical compositions may conveniently be administered in unit dosage form and may be prepared by dissolving, dispersing or otherwise combining an active compound as defined above and optional pharmaceutical adjuvants or other agents in a carrier such as, for example, water, saline, aqueous dextrose, glycerol, ethanol, and the like, to thereby form a solution or suspension. Actual methods of preparing such dosage forms are well-known in the pharmaceutical art, for example as described in Remington's Pharmaceutical Sciences, Mack Publishing Company, Easton, PA., 1970. Formulations for parenteral administration may also contain as common excipients polyalkylene glycols such as polyethylene glycol, oils of vegetable origin, hydrogenated naphthalenes and the like. U.S. Patent No. 4,389,414 describes parenteral pharmaceutical compositions which are well suited to administration of prostaglandin analogs.

Formulations for vaginal or rectal administration, e.g. suppositories, may contain as excipients, for example, polyalkyleneglycols, vaseline, cocoa butter, and the like. For buccal administration typical excipients include sugars, calcium stearate, magnesium stearate, pregelatinated starch, and the like.

For solid compositions such as oral dosage forms, conventional non-toxic carriers include, for example, pharmaceutical grades of mannitol, lactose, starch, magnesium stearate, sodium saccharin, talcum, cellulose, glucose, sucrose, magnesium carbonate, and the like. The active agents, as defined above, may also be formulated in solid sustained release formulations, including tablets, injectable microcapulses and other semi-solid sustained release injectable formulations, osmotic delivery systems, biocompatible and/or biodegradable implants, and the like, available in the pharmaceutical arts.

A dosage form may contain a pharmaceutically accepable non-toxic salt of the active compound which has a low degree of solubility in body fluids, for example, (a) an acid addition salt with a polybasic acid such as phosphoric acid, sulfuric acid, citric acid, tartaric acid, tannic acid, alginic acid, polyglutamic acid, naphthalene mono-or di-sulfonic acids, polygalacturonic acid, and the like; (b) a salt with a polyvalent metal cation such as zinc, calcium bismuth, barium, magnesium, aluminum, copper, cobalt, nickel, cadmium and the like, or with an organic cation formed from e.g., N,N'-dibenzylethylenediamine or ethylenediamine; or (c) combinations of (a) and (b) e.g. a zinc tannate salt.

The LHRH antagonist containing pharmaceutical compositions for use in this invention generally contain from 20 μg/ml to 50 mg/ml, preferably from 100 μg/ml to 10 mg/ml of the LHRH antagonist compound. Prostaglandin analog-containing pharmaceutical compositions for use in the invention will generally contain from 1 μg/ml to 1 mg/ml, preferably 25 to 100 μg/ml. These concentration ranges are given for compounds within the potency range of the the preferred compounds for use in this invention. If LHRH antagonists and prostaglandin analogs having lower potencies are used, a concentration of active agent in a particular pharmaceutical composition greater than that stated herein may be required, and is included within the scope of this invention. The following Examples further illustrate the invention, and are not to be construed as in any way limiting the scope of the invention as described in the Summary, the Detailed Description and in the appended claims.

## EXAMPLE 1

### Termination of pregnancy with an LHRH Antagonist

A bitch was checked daily for sexual receptivity and was allowed to breed with a stud male each day until she was no longer receptive. Vaginal cytology was monitored daily to identify the first day fo diestrus (first day of gestation). Plasma progesterone concentration was monitored; the levels of progesterone at 8 and 4 days before the first day of diestrus were 4 and 6 ng/ml, respectively. On the first day of diestrus the plasma progesterone concentration had risen to 30 ng/ml and continued to rise to a peak on day 9 of gestation, slowly decreasing thereafter to 30 ng/ml on day 25 of gestation. Beginning on day 25 of gestation, the bitch received daily subcutaneous injections of 300 μg/kg of [N-Ac-D-Nal(2)[1], D-p-Cl-Phe[2], D-Trp[3],D-Deh[6], D-Ala[10]]LHRH for 7 days. After the first injection the plasma progesterone level immediately dropped to 4 ng per milliliter. At the end of the course of treatment, the level of plasma progesterone was below 1* nanogram per milliliter. A fetus was expelled on the 5th day of treatment and a tissue mass was expelled on day 49, which was 24 days after the start of treatment.

## EXAMPLE 2

Effect of time of dosing with two different LHRH antagonists on pregnancy in bitches

Several pregnant beagle bitches were treated with one of two different LHRH antagonists administered subcutaneously. In Part A the dogs received daily injections of the LHRH antagonists during the days shown. In Part B the dogs received continuous subcutaneous infusion over the period of days shown. The results are summarized in Table 1.

9

## Table 1

| DOG # | TREATMENT ON DAYS OF PREGNANCY | COMMENT |
|---|---|---|
| Part A. Treatment with $[\text{N-Ac-D-Nal}(2)^1$, D-p-Cl-Phe$^2$ ,D-Trp$^3$, D-Deh$^6$, D-Ala$^{10}]$-LHRH | | |
| 1 | 36-42 | Aborted on days 39-41 of pregnancy |
| 2 | 32-38 | Aborted on day 35 of pregnancy |
| Part B. Treatment with $[\text{N-Ac-D-Nal}(2)^1$, D-p-Cl-Phe$^2$, D-Pal$(3)^3$,D-Deh$^6$, D-Ala$^{10}]$LHRH | | |
| 3 | 29-35 | Resorbed, discharge day 35 |
| 4 | 28-34 | Resorbed, discharge day 34 |

EXAMPLE 3

Termination of pregnancy with a single dose of LHRH Antagonist

A beagle bitch was bred and confirmed to be pregnant by manual palpation of the uterus during the fourth week of gestation. On the twenty-ninth day of pregnancy she was administered a single subcutaneous injection of an aqueous solution of 50 mg of [N-Ac-D-Nal(2)$^1$, D-p-Cl-Phe$^2$, D-Trp$^3$, D-Deh$^6$, D-Ala$^{10}$]-LHRH. Immediately before injection the plasma progesterone level was 19ng/ml and by 24 hours after injection this had declined to 3.3 ng/ml. Plasma levels of progesterone varied thereafter from day to day but were never above 6 ng/ml. Delivery of an abortus was noted on the 36th day of pregnancy.

EXAMPLE 4

Treatment with LHRH antagonist to terminate pregnancy during very early pregnancy

A beagle bitch was mated and treated on day 1 of gestation with a single subutaneous injection of 10 mg/kg of [N-Ac-D-Nal(2)$^1$,D-p-Cl-Phe$^2$,D-Trp$^3$D-Deh$^6$-D-Ala$^{10}$]LHRH. The bitch was confirmed to be pregnant by manual palpation on day 19 of gestation. The bitch had a heavy sanguineous vaginal discharge on days 24-34 of gestation. At expected term, no signs of delivery were noted and the bitch was confirmed to be not pregnant by abdominal palpation.

EXAMPLE 5

Effect of combination treatment with LHRH antagonist and PG analog to terminate pregnancy

A. Treatment on days 1 or 2 of gestation

Several beagle bitches were mated and treated on day 1 or 2 of gestation with a low dose of [N-Ac-D-Nal(2)[1],D-p-cl-Phe[2], D-Trp[3],D-Deh[6],D-Ala[10]]LHRH alone, a low dose of d,1 9α,11α,15α-trihydroxy-16-phenoxy-17,18,19,20-tetranorprosta-4,5,13-transtrienoic acid n-propyl ester alone, or with a combination of the same low doses of both agents. The results are summarized in Table 2.

## TABLE 2

| No. of Dogs | Dose of LHRH antagonist | Dose of prostaglandin | Continuation of Pregnancy |
|---|---|---|---|
| 1 | 2mg/kg | - | Yes |
| 1 | - | 20 μg/kg | Yes |
| 3 | 2mg/kg | 20 μg/kg | No |

B. Treatment during the first 8 days of gestation

Several beagle bitches were mated and treated on days 2 or 8 of gestation with [N-Ac-D-Nal(2)[1],D-p-cl-Phe[2], D-Trp[3],D-Deh[6],D-Ala[10]]LHRH alone, or the LHRH antagonist together with d,1 9α,11α,15α-trihydroxy-16-phenoxy-17,18,19,20-tetranorprosta-4,5,13-transtrienoic acid n-propyl ester. Daily measurements of plasma progesterone levels were made which confirmed that the combination treatment terminated pregnancy by decreasing the progesterone to levels incapable of maintaining gestation, as shown in Table 3.

## Table 3

Effect of subcutaneous injection of LHRH antagonist*
and prostaglandin analog** on circulating levels
of progesterone and outcome of gestation in
pregnant beagle bitches

| Dog | Day of dosing (Day of gestation) | Dose of | | Progesterone levels (ng/kg) before and after day of dosing (day 0) | | | | | | | | Continuation of Pregnancy |
|-----|------|-------------|----------|-----|----|----|----|----|----|----|----|------|
| | | LHRH analog | PG analog | -2 | 0 | 1 | 2 | 3 | 4 | 5 | 6 | |
| 1 | 2 | 2mg/kg | 20µg/kg | 56 | 13 | 7 | 5 | 4 | 5 | 4 | 5 | No |
| 2 | 2 | 2mg/kg | 20µg/kg | 43 | 45 | 15 | 3 | 4 | 5 | 4 | 3 | No |
| 3 | 8 | 2mg/kg | 20µg/kg | 29 | 40 | 7 | 2 | 1 | 2 | 2 | 2 | No |
| 4 | 8 | 2mg/kg | 20µg/kg | 23 | 19*. 5 | 4 | 4 | 4 | 4 | 4 | | No |
| 5 | 2 | 2mg/kg | - | 52 | 54 | 34 | 20 | 25 | 52 | 48 | 23 | Yes |
| 6 | 2 | 2mg/kg | - | 47 | 55 | 26 | 20 | 20 | 32 | 10 | 8 | Yes |

\*     [N-Ac-D-Nal(2)[1],D-p-Cl-Phe[2],D-Trp[3]-D-Deh[6],D-Ala[10]]LHRH.

\*\*    d,l,-9$\alpha$,11$\alpha$,15$\alpha$-trihydroxy-16-phenoxy-17,18,19,20-tetranorprosta-4,5,13-transtrienoic acid n-propylester.

EXAMPLE 6

Termination of estrus with an LHRH antagonist

Beagle bitches were followed closely until a slight sanguineous vaginal discharge could be identified, (first day of pro-estrus) which marked Day 1 of the experiment. Microscopic evaluation of vaginal cytology was used to diagnose pro-estrus. The dogs were then treated, starting on one of days 1-3 of pro-estrus, by a single s.c. injection, or by multiple consecutive daily s.c. injections of [N-Ac-D-Nal(2)$^1$,D-p-Cl-Phe$^2$,D-Trp$^3$,D-Deh$^6$,D-Ala$^{10}$]LHRH. Vaginal cytology was followed daily for the presence of cornified cells which show the bitches to be entering estrus (heat). The vaginal discharge rapidly diminished in quantity and within 2 days the bitches typically had reentered both behavioral and cytological diestrus. The bitches showed minimal to no sexual receptivity and usually would not allow mating by stud males. On the two occasions when a mating did occur, ovulation and pregnancy were still prevented. (Dogs typically show several days to a week or more when they are sexually receptive and will mate daily or more frequently during this time). Some bitches were treated again when heat returned, with similar results. All bitches bred at the return heat whether they had received one or more courses of treatment, and conceived and produced normal offspring. The results of this experiment are summarized in Table 4.

## Table 4

Estrus termination activity of subcutaneously injected LHRH antagonist* when given at onset of vaginal bleeding in beagle bitches

| Dose | # Doses | # Not Showing Estrus Behavior | Number Bitches Ovulating | Interval to Next Heat | Pregnant Next Cycle |
|---|---|---|---|---|---|
| 300 µg/kg | 7 | 1/1 | 0/1 | 27 days | – |
| 300 µg/kg | 14 | 3/3 | 0/3 | 40 + 60 (days) | 1/1 |
| 2 mg/kg | 1 | 7/9 | 0/14[a] | 26 + 1 (days) | 6/6 |

a   animals were treated again on the return heat.

*   [N-Ac-D-Nal(2)$^1$,D-p-Cl-Phe$^2$,D-Trp$^3$,D-Deh$^6$, D-Ala$^{10}$]LHRH

EXAMPLE 7

Contraceptive effect of subcutaneous injection of an LHRH antagonist co-administered with a prostaglandin analog to a bitch within 2 days after mating

A beagle bitch is diagnosed as entering proestrus from detection of a sanguineous vaginal discharge (Day 1 of experiment). The bitch is then exposed daily to a male of proven fertility. For several days the bitch refuses the sexual overtures of the male, by either snapping at him or sitting down. After this time the bitch begins to signal that she has entered a period of sexual receptivity by "flagging" and by direct overtures to the male. She stands to being mounted, permits intromission and ejaculation and remains "tied" to the male for at least 20 minutes. Two days after mating the bitch is given, at separate sites, a subcutaneous injection of 2 mg/kg of [N-Ac-D-Nal(2)[1],D-p-Cl-Phe[2], D-Trp[3],D-Deh[6],D-Ala[10]]LHRH, and a subcutaneous injection of a mg/kg of d.1 $9\alpha,11\alpha,15\alpha$-trihydroxy-16-phenoxy-17,18,19,20-tetranorprosta-4,5,13-transtrienoic acid n-propylester. Measurement of plasma levels of progesterone in blood samples taken from Day 1 of the experiment onward shows that progesterone levels decline following treatment and are maintained at low levels (< 5 ng/ml) for 12 days. Pregnancy is not detected at palpation on day 20 of gestation (20 days of diestrus). At normal term no signal of delivery are noted and abdominal palpation confirms lack of pregnancy.

EXAMPLE 8

Lack of effect on gestation of subcutaneous administration of an LHRH antagonist to pregnant cats

Pregnant cats were bred and 15-18 days later were implanted subcutaneously with an Alzet® minipump filled with a solution of [N-Ac-D-Nal(2)[1], D-p-Cl-Phe[2], D-Trp[3], D-Deh[6],D-Ala[10]]LHRH in propylene glycol at a concentration sufficient to deliver 300 $\mu$g/kg/day. The pumps were removed after seven days. No effect of treatment on circulating levels of progesterone could be discerned in daily plasma samples. All treated cats continued to normal uneventful term delivery. The plasma levels of progesterone before and after treatment are shown in Table 5.

## Table 5

Effect of 300 μg/kg/day of [N-Ac-D-Nal(2)[1], D-p-Cl-Phe[2],D-Trp[3], D-Deh[6],D-Ala[10]]LHRH for seven days on plasma levels of progesterone in pregnant cats.

| Days from Treatment | Plasma level of progesterone (ng/ml) | |
|---|---|---|
| | Cat 1 | Cat 2 |
| -2 | 4.5 | 4.3 |
| -1 | 2.0 | 3.0 |
| 0 | 4.0 | 2.3 |
| 1 | 2.7 | 4.1 |
| 2 | 4.3 | 5.2 |
| 3 | 4.0 | 3.9 |
| 4 | 4.4 | 4.2 |
| 5 | 3.8 | 4.2 |
| 6 | 5.7 | 6.3 |
| 7 | 3.3 | 5.5 |
| 8 | 6.5 | 6.1 |
| 9 | 6.7 | 7.3 |
| 10 | 5.2 | 6.7 |

This test shows that no significant effects were obtained in female cats administered the LHRH-antagonist.

### EXAMPLE 9

Treatment of pyometritis by subcutaneous administration of an LHRH antagonist and a prostaglandin analog

A bitch has presented with an opaque vaginal discharge, containing an abnormally high level of leukocytes, approximately 1.5 months after an estrous episode. The uterus is enlarged as judged by abdominal palpation, and pyometritis is diagnosed. The bitch is given single subcutaneous bolus injections of 2 mg/kg of [N-Ac-D-Nal(2)[1],D-p-Cl-Phe[2],D-Trp[3]D-Deh[6], D-Ala[10]]LHRH, and 20 μg/Kg of d,l 9α,11α,15α-trihydroxy-16-phenoxy-17,18,19,20-tetranorprosta 4,5,13-transtrienoic acid n-propylester at two separate sites. The animal is not hospitalized. Ampicillin daily injection is started and continued for 7 days to guard against secondary infection. Within 1 hour after administration of the LHRH antagonist and prostaglandin analog an increase in the vaginal discharge is noted which continues during the next 4 days. Uterine size decreases over this time period. Assays of blood samples show a precipitous decline in circulating levels of progesterone over the first several hours after treatment, which remain at less than 1 ng/ml for the duration of the study. Four months later, the bitch comes into heat, is bred and produces a normal litter.

EXAMPLE 10

Typical pharmaceutical compositions contain as active ingredient an LHRH antagonist, for example N-Ac-D-Nal(2)-D-p-CL-Phe-D-Trp-Ser-Tyr-D-Deh Leu-Arg-Pro-D-AlaNH$_2$, by itself or as a pharmaceutically acceptable salt, e.g. the acetic acid addition salt, the zinc salt, the zinc tannate salt, etc.

The following is an example of a composition containing the acetate salt:

---

## Subcutaneous Injectable Solution

| | |
|---|---|
| LHRH Antagonist, acetate salt | 10.0 mg |
| Benzyl Alcohol | 9.0 mg |
| Glacial Acetic Acid | 1.2 mg |
| Propylene Glycol | 200.0 mg |
| Mannitol | 35.0 mg |
| Sterile Water for injection | 1.0 ml |

---

Method of Manufacture

A. Preparation of Vehicle:

Benzyl alcohol is dissolved in approximately 50% of the water, at about 40°C. Propylene glycol and mannitol are each successively dissolved in this solution, which is maintained at 40°C. The resulting solution is cooled to room temperature and acetic acid is added. The pH of the solution is adjusted to 5.0 ± 0.2 with 1.0 N NaOH, and brought to volume with sterile water.

B. Preparation of Active Composition:

The LHRH antagonist is dissolved in 90% of the prepared vehicle, and the pH is adjusted to 5.0 ± 0.2 with 1.0 N NaOH. The volume is brought up with the vehicle, the solution is mixed and filtered through a suitable filter. Then, 5 ml clear type-I multiple-dose vials are aseptically filled with 5.2 ml of the final solution, stoppered and sealed, and autoclaved for sterility.

EXAMPLE 11

This example sets forth the process for preparing an injectable solution of d,1 9α,11α,15α-trihydroxy-16-phenoxy-17,18,19,20-tetranorprosta-4,5,13-transtrienoic acid n-propylester. Other suitable prostaglandin analogs as described above for use in this invention can be substituted as the active compound in this example.

Two and a half grams (g) of d,1 9α,11α,15α-trihydroxy-16-phenoxy-17,18,19,20-tetranorprosta-4,5,13-transtrienoic acid n-propylester, 2.5 g ascorbic acid (antioxidant) and 45 g benzyl alcohol (preservative) were dissolved with stirring in polyethylene glycol 400 to prepare 5.0 liters of the composition. The resulting solution was then sterile filtered through a 0.4 micron membrane filter. The sterile solution was then aseptically sterile filled into sterile vials and sealed. Each 1 ml vial contains 0.5 mg active compound.

EXAMPLE 12

An injectable solution for co-administration of a combination of an LHRH antagonist and a prostaglandin analog is prepared by combining, and mixing by shaking, the contents of a vial containing an appropriate dosage of an LHRH antagonist in an injectable solution prepared as described in Example 10, with a vial containing an appropriate dosage of a prostaglandin analog in an injectable solution prepared as described in Example 11. The vials are combined just prior to administration to the dog of the mixed solution via a single bolus injection.

**Claims**

1. The use of an LHRH antagonist in the manufacture of a medicament for terminating pregnancy in female dogs.

2. The use of an LHRH antigonist in the manufacture of a medicament for terminating estrus and heat in a dog.

3. The use of an LHRH antagonist in the manufacture of a medicament for controlling the time of estrus in a dog.

4. The use of an LHRH antagonist in the manufacture of a medicament for treating hormonally mediated uterine infections in a female dog.

5. The use of a combination of an LHRH antagnist and a prostaglandin analog in the manufacture of a medicament for terminating pregnancy in female dogs.

6. The use of a combination of an LHRH antagonist and a prostaglandin analog, in the manufacture of a medicament for treating hormonally mediated uterine infections in a female dog.

7. The use of any one of claims 1 to 6 wherein the LHRH antagonist is [N-Ac-D-Nal(2)$^1$,D-p-Cl-Phe$^2$, D-Trp$^3$,D-Deh$^6$,D-Ala$^{10}$]LHRH or a pharmaceutically acceptable salt thereof.

8. The use of any one of claims 5 to 7 wherein the prostaglandin analog is d,1 $9\alpha,11\alpha,15\alpha$-trihydroxy-16-phenoxy-17,18,19,20-tetranorprosta-4,5,13-transtrienoic acid or an alkyl ester thereof having 1-12 carbon atoms, preferably d,1 $9\alpha,11\alpha,15\alpha$-trihydroxy-16-phenoxy-17,18,19,20-tetranorprosta-4,5,13-transtrienoic acid n-propylester.

9. A pharmaceutical composition for terminating pregnancy in a female dog which comprises an effective amount of an LHRH antigonist, or a pharmaceutically acceptable salt thereof, in admixture with at least one pharmaceutically acceptable excipient, wherein such amount is designed to be delivered as a) a single dose of an LHRH antagonist in the range of about 8-20 mg/kg, at any time during pregnancy, or b) a single dose of an LHRH antagonist in the range of about 0.2-6mg/kg, on or after about 14 days of gestation, or c) daily or continuous doses beginning on or after day 14 of gestation over a period of about 4-8 days, said daily dose being in the range of about 50-350 $\mu$g/Kg/day.

10. A pharmaceutical composition for terminating pregnancy which comprises low and independently ineffective amounts of an LHRH antagonist, or pharmaceutically acceptable salt thereof, and a prostaglandin analog, in admixture with at least one pharmaceutically acceptable excipient.

11. A pharmaceutical composition for terminating estrus in dogs which comprises an effective amount of an LHRH antagonist, or a pharmaceutically acceptable salt thereof, in admixture with at least one pharmaceutically acceptable excipient, wherein such amount is designed to be delivered as dialy or continuous doses in the range of about 50-350 $\mu$g/Kg/day for a period of 7-14 days.

12. A pharmaceutical composition for controlling the time of estrus which comprises an effective amount of an LHRH antagonist, or a pharmaceutically acceptable salt thereof, in admixture with at least one pharmaceutically acceptable excipient.

13. A pharmaceutical composition for treating a hormonally mediated uterine infection in a female dog which comprises an effective amount of an LHRH antagonist, or pharmaceutically acceptable salt thereof, in admixture with at least one pharmaceutically acceptable excipient, wherein such amount is designed to be delivered as a) a single dose of an LHRH antagonist in the range of about 8-20 mg/kg at any time during pregnancy, or b) a single dose of an LHRH antagonist in the range of about 0.2-6mg/kg, on or after about 14 days of gestation, or c) daily or continuous doses beginning on or after day 14 of gestation over a period of about 4-8 days, said daily dose being in the range of about 50-350 $\mu$g/Kg/day.

14. A pharmaceutical composition for treating a hormonally mediated uterine infection in a female dog which comprises low and independently ineffective amounts of an LHRH antagonist, or pharmaceutically acceptable salt thereof, and a prostaglandin analog, in admixture with at least one pharmaceutically acceptable excipient.

15. An LHRH antagonist for use in terminating pregnancy in dogs.

16. An LHRH antagonist for use in terminating estrus and heat in a dog.

17. An LHRH antagonist for use in controlling the time of estrus in a dog.

18. An LHRH antagonist for use in treating hormonally mediated uterine infections in a female dog.